# EUROPÄISCHE PATENTANMELDUNG

(11) **EP 2 742 911 A1**
(43) Veröffentlichungstag der Anmeldung: **18.06.2014**
(21) Anmeldenummer: 12008390.2
(22) Anmeldetag: 17.12.2012
(51) Int. Cl.: A61F 2/24

(54) **Klappenprothese zum Ersatz einer Atrioventricularklappe**

(71) Anmelder: Figulla, Hans Reiner, 07749 Jena (DE); Lauten, Alexander, 07743 Jena (DE)
(72) Erfinder: Figulla, Hans Reiner, 07749 Jena (DE); Lauten, Alexander, 07743 Jena (DE)
(74) Vertreter: KIPA AB

(57) **Zusammenfassung**

Eine Klappenprothese zum Ersatz einer Atrioventricularklappe des Herzens hat einen Doppelringkörper (10), an dem Herzklappensegel befestigt sind und der einen ventrikelseitigen Ring (12) und einen atriumseitigen Ring (14) aufweist, wobei der ventrikelseitige Ring ovale Form hat und mit Ankern (28) versehen ist.

## Beschreibung

Die Erfindung betrifft eine Klappenprothese zum Ersatz einer Atrioventricularklappe des Herzens, also der Mitralklappe oder der Trikuspidalklappe.

Die WO 2012/095116 A1 beschreibt eine Klappenprothese zum Ersatz einer Atrioventricularklappe des Herzens mit einem Ringkörper, von dem sich Verankerungsteile axial so erstrecken, dass sie durch Drehung des Ringkörpers in verankernden Eingriff mit Gewebe, wie z.B. Sehnenfäden, bringbar sind.

Bei Patienten mit einer Funktionsbeeinträchtigung einer Herzklappe ist ein offener chirurgischer Eingriff am Herzen zum Einsatz einer Klappenprothese (Ersatzklappe) aufgrund des Allgemeinzustandes des Patienten häufig mit erhöhten Risiken verbunden, sodass Herzklappenprothesen zunehmend minimal invasiv über einen Katheter implantiert werden.

Der Stand der Technik kennt hierfür den Einsatz von Stents, in denen Ersatz-Herzklappensegel befestigt sind und die zur Einführung über einen Katheter stark komprimierbar sind und so durch den Katheter an den Ort der zu ersetzenden Herzklappe vorschiebbar und dort freisetzbar sind. Der zum Beispiel ballonexpandierbare oder selbstexpandierbare Stent entwickelt im freigesetzten Zustand eine radiale Spreizkraft, die eine Verankerung der Ersatz-Klappenprothese bewirkt oder zumindest fördert. Für eine solche Verankerung der Klappenprothese ist eine Ersatz-Aortenklappe besonders geeignet, die mit radialer Spreizkraft am Ort der dysfunktionalen Aortenklappe verankert werden kann. Zum Stand der Technik hierzu vgl. z.B. EP 1 994 913 A2; EP 1 469 797 B1; EP 1 259 195 B1; WO 2007/051620 A1; WO 2007/048529 A1; EP 1 980 220 A1; WO 01/64137 A1; EP 1 255 510 B3; und US 5,411,552.

Die Mitralklappe des Herzens, also die Klappe zwischen dem linken Vorhof (Atrium) und der linken Kammer (Ventrikel) ist allerdings wenig geeignet für eine Ersatzprothese, die wesentlich durch radiale Spreizkraft eines Stents kraftschlüssig (durch Reibung) vor Ort verankert ist, da eine Aufweitung des Annulus zu vermeiden ist.

Der Erfindung liegt die Aufgabe zu Grunde, eine Klappenprothese zum Ersatz einer Atrioventricularklappe bereitzustellen, die mittels eines Katheters implantierbar ist und eine stabile sowie orthotrope Positionierung und Verankerung ermöglicht, wobei die Blutströmung im Herzen möglichst wenig behindert sein soll, insbesondere die Blutströmung durch einen Ausflusstrakt.

Eine erfindungsgemäße Klappenprothese zum Ersatz einer Atrioventricularklappe des Herzens ist versehen mit einem Doppelringkörper, an dem Herzklappensegel befestigt sind und der einen ventrikelseitigen Ring und einen atriumseitigen Ring aufweist, wobei der ventrikelseitige Ring auf seinem äußeren Umfang nur in ausgewählten Sektoren mit im Wesentlichen radial nach außen stehenden Ankern versehen ist, während zumindest ein Sektor des Ringes von derartigen Ankern frei bleibt. Von Ankern frei bleibt derjenige Sektor des Ringes, der bei bestimmungsgemäßem Einsatz der Klappenprothese im Herzen im Bereich des Ausflusstraktes liegt. Bei z.B. einem Einsatz der Klappenprothese zwischen dem linken Vorhof und der linken Herzkammer ist der genannte Ausflusstrakt der Zugang zur Aorta. Die genannten Anker, die der Abstützung der Klappenprothese im Bereich des Annulus zwischen der Vorkammer und der Kammer dienen, sind also so angeordnet, dass die Blutströmung aus der Kammer in den Ausflusstrakt (z.B. die Aorta bzw. die Lungenarterie) möglichst frei bleibt von Draht-Hindernissen. Solche Hindernisse verringern nicht nur den Blutdurchsatz, sie sind auch aus biochemischen Gründen aus Bereichen starker Blutströmung herauszuhalten.

Eine bevorzugte Variante der Erfindung sieht vor, dass an einem Doppelringkörper, der mit Herzklappensegeln versehen ist, ventrikelseitig und atriumseitig jeweils ein Ring oder Wulst ausgebildet ist, wobei der ventrikelseitige Ring, bezogen auf seine Mittelachse, nicht perfekt kreisförmig ist, sondern in zumindest einem Sektor einen reduzierten Durchmesser aufweist. Dieser Sektor mit reduziertem Durchmesser wird bei Einsatz der Klappenprothese im Herzen so positioniert, dass er im Bereich des Zuflusses zu einem Ausflusstrakt (z.B. Aorta bzw. Lungenarterie) liegt derart, dass die Blutströmung in den Ausflusstrakt der Kammer möglichst wenig behindert wird, während die Bereiche des ventrikelseitigen Ringes mit größerem Durchmesser der Abstützung der Klappenprothese in anderen Bereichen ventrikelseitig des Annulus dienen.

Der Ring kann bei allen Ausführungsbeispielen auch eine Ringscheibe sein.

Dabei sind bei allen Ausführungsbeispielen der Erfindung die genannten Anker bzw. die genannte Abstützung an ihren mit Herzgewebe in Eingriff kommenden Enden abgerundet, beispielsweise sind die Anker schlaufenförmig. Die Anker bzw. die genannte Abstützung sind bevorzugt im Wesentlichen (in Bezug auf die Mittelachse der Prothese) radial nach außen gerichtet, wobei sie auch in axialer Richtung zum Annulus hin abgewinkelt sein können, so dass die Anker bzw. die Abstützung eine Hakenform haben mit spitzem Winkel zwischen Anker und Mittelachse der Prothese.

Bevorzugt hat die Klappenprothese allgemein etwa diaboloförmige Gestalt mit einem axial etwa in der Mitte liegenden Bereich reduzierten Durchmessers, der so ausgestaltet ist, dass er bei Einsatz zwischen Atrium und Ventrikel im Bereich des Annulus positionierbar ist.

Eine Ausführungsform der Erfindung sieht vor, dass der Doppelringkörper adaptiert ist, bei Einsatz im Klappenannulus des Herzens keine den Klappenannulus stark aufweitende radiale Spreizkraft zu erzeugen. Eine geringe Speizkraft, die den Doppelringkörper im Klappenannulus zentriert ist gleichwohl förderlich.

Andere Ausgestaltungen der Erfindung sehen vor, dass der Doppelringkörper aus einem Geflecht geformt ist, insbesondere aus einem Material mit Formgedächtnis.

Der Doppelringkörper kann ballonexpandierbar sein oder selbstexpandierbar.

Wenn im Zusammenhang mit der erfindungsgemäßen Klappenprothese von einer "ovalen" Form die Rede ist, dann bedeutet dies funktional, dass das betreffende Bauteil, z.B. der genannte Ring, derart gestaltet ist, dass das Bauteil bzw. der Ring zumindest einen Bereich mit reduziertem Durchmesser aufweist, der im Einsatz so im Bereich des Ausflusstraktes der Herzkammer (z.B. Aorta oder Lungenarterie) anzuordnen ist, dass in diesem Bereich die Blutströmung möglichst wenig durch Komponenten der Klappenprothese behindert ist. Somit kann der Ring nicht nur oval im engeren Sinn des Wortes sein, sondern auch "einseitig" oval, also etwa nierenförmig.

Nachfolgend wird ein Ausführungsbeispiel der Erfindung anhand der Zeichnung näher erläutert. Es zeigt:
- Figur 1: eine perspektivische Seitenansicht einer Klappenprothese,
- Figur 2: eine Draufsicht auf die Klappenprothese gemäß Figur 1 in Richtung der Mittelachse A₁ der Klappenprothese,
- Figur 3: eine Klappenprothese gemäß den Figuren 1 und 2 in situ im Herzen; und
- Figur 4: eine axiale Draufsicht auf die Klappenprothese.

Die in den Figuren gezeigte Klappenprothese zum Ersatze einer Atrioventricularklappe des Herzens weist einen Doppelringkörper 10 auf, in dem Herzklappensegel befestigt sind. Für die Herzklappensegel können als solches bekannte Systeme des Stands der Technik eingesetzt werden, z.B. Segel aus Pericardium, oder z.B. vom Herz eines Schweines gewonnene Herzklappen, die in dem Doppelringkörper vernäht werden. Eine solche Anordnung von Herzklappensegeln in einem stentartigen Gebilde sind als solches bekannt.

Der Doppelringkörper 10 ist aus einem Drahtgeflecht gebildet und die elastischen Eigenschaften des Drahtes sowie die Abmessungen des Doppelringkörpers 10 sind so gewählt, dass der Doppelringkörper im Einsatz zum Ersatz z.B. einer Mitralklappe oder einer Trikuspidalklappe - anders als üblicherweise ein Stent - keine den natürlichen Klappenannulus wesentlich aufweitende radiale Spreizkraft erzeugt sondern nur eine geringe, eine Zentrierung des Doppelringkörpers 10 im Annulus bewirkende Spreizkraft.

Für das Drahtgeflecht des Doppelringkörpers 10 kann z.B. ein Metall mit Formgedächtnis eingesetzt werden, wie Nitinol.

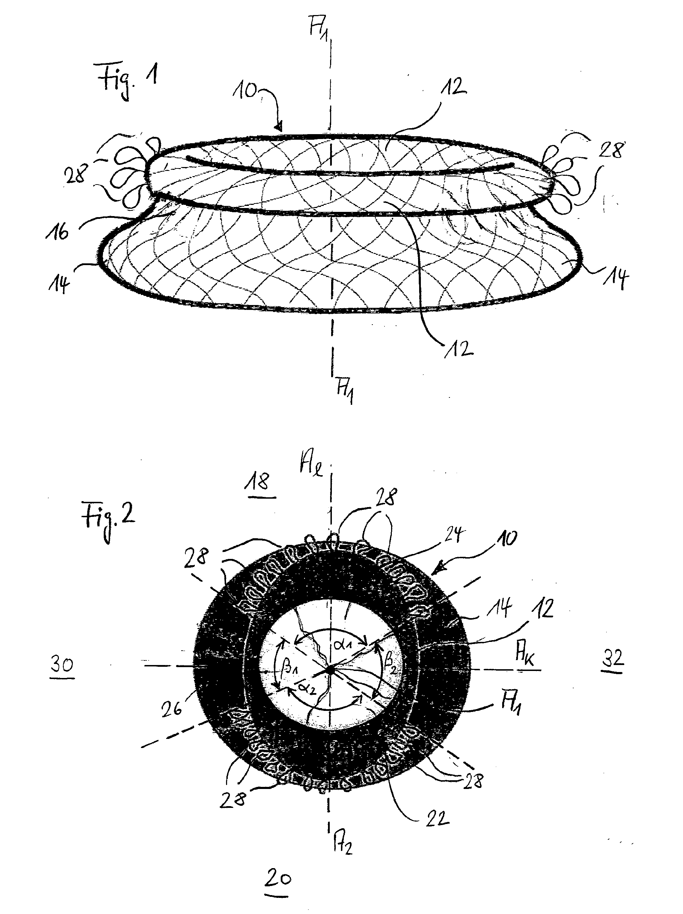

Wie insbesondere Figur 1 zeigt, hat der Doppelringkörper 10 zwei Wülste, die hier bezeichnet werden als ventrikelseitiger Ring 12 und atriumseitiger Ring 14. Die Bezeichnungen "ventrikelseitig" bzw. "atriumseitig" beziehen sich auf die Positionierung der Prothese im Herzen.

Zwischen dem ventrikelseitigen Ring 12 und dem atriumseitigen Ring 14 hat der Doppelringkörper 10 einen mittleren Bereich 16, dessen Durchmesser geringer ist als der Durchmesser der beiden genannten Ringe. Dieser mittlere Bereich 16 kommt beim Einsatz im Herzen im Annulus zwischen den beiden Herzkammern zu liegen, ohne dabei den Annulus wesentlich aufzuweiten.

Figur 2 zeigt eine Draufsicht auf den Doppelringkörper 10 gemäß Figur 1 aus Richtung von dessen Mittelachse A₁, in Figur 1 von oben. Entsprechend zeigt Figur 1 eine Ansicht des Doppelringkörpers 10 gemäß Figur 2 von der Seite in Figur 2, also z.B. von rechts in der Zeichnungsebene.

Gemäß der Draufsicht in Figur 2 ist der atriumseitige Ring 14 etwa kreisförmig, während der ventrikelseitige Ring 12 etwa oval geformt ist.

Bezogen auf den Einbauzustand im Herzen kann in Figur 2 der Bereich 18 mit "oben" bezeichnet werden und der Bereich 20 mit "unten".

In der Draufsicht gemäß Figur 2 sind drei Herzklappensegel, die im Inneren des Doppelringkörpers 10 angebracht sind, mit dem Bezugszeichen 22, 24, 26 versehen.

Gemäß den Figuren 1 und 2 weist der ventrikelseitige Ring 12 Anker 28 auf. Diese Anker 28 erstrecken sich nicht vollständig äquidistant über den vollen Umfang des ventrikelseitigen Ringes 12 sondern sind auf Sektoren des Ringes beschränkt. Diese Sektoren sind in Figur 2 mit den Winkeln α₁ und α₂ gekennzeichnet. Entsprechend sind andere Sektoren des etwa oval gestalteten ventrikelseitigen Ringes 14 frei von Ankern, im Ausführungsbeispiel gemäß Figur 2 sind dies die mit den Winkeln β₁ und β₂ gekennzeichneten Sektoren.

Bei Einsatz im Herzen wird die Klappenprothese so positioniert, dass ein von Ankern 28 freier Sektor (also ein Sektor gemäß einem der Winkel β₁ oder β₂) im Bereich eines Ausflusstraktes (nicht gezeigt) des Herzens positioniert wird, so dass kein Anker 28 im Bereich der Blut-Hauptströmung liegt, die in den Ausflusstrakt der Kammer führt. Bei Einsatz der Klappenprothese zwischen linker Kammer und linkem Vorhof ist der Ausflusstrakt der Eingang zur Aorta. Dieser linksventrikuläre Ausflusstrakt befindet sich z.B. in Figur 2 auf der Seite, die dort mit dem Bezugszeichen 30 gekennzeichnet ist. Das Bezugszeichen 32 kennzeichnet somit die Lage der Lateralwand.

Wie Figur 2 zeigt, hat bei diesem Ausführungsbeispiel der ventrikelseitige Ring 12 eine lange Achse Aₗ und eine kurze Achse Aₖ. Ein Sektor des ventrikelseitigen Ringes 12, der sich gemäß dem Winkel β₁ beidseitig der kurzen Achse Aₖ erstreckt, ist frei von Ankern 28, um die Blutströmung im Bereich 30 des linksventrikulären Ausflusstraktes möglichst frei von Drahthindernissen oder dergleichen zu halten. Beim Ausführungsbeispiel nach Figur 2 ist auch der gegenüberliegende Sektor gemäß dem Winkel β₂ frei von Ankern. Dies erfolgt beim dargestellten Ausführungsbeispiel zur Erleichterung der Positionierung der Klappenprothese im Herzen, da es bei dieser Ausgestaltung zwei Positionen gibt, in denen das vorstehend genannte Ziel des Freihaltens des Ausflusstraktes von Hindernissen durch Drehung der Prothese im ihre Achse A₁ mit zwei Stellungen erreichbar ist.

Die Anker 28 stützen, im Zusammenwirken mit dem ventrikelseitigen und dem atriumseitigen Ring die Klappenprothese im Annulus ab. Dabei können die im dargestellten Ausführungsbeispiel schlaufenförmigen Anker 28 (etwas abweichend von der Zeichnung) so hakenförmig (in Figur 1 nach unten) gebogen sein, dass sie bis annähernd zum ventrikelseitigen Bereich des Annulus reichen und sich dort abstützen, so dass im Schließzustand der Klappenprothese der ventrikelseitige Druck aufgefangen wird.

Bei dieser Ausgestaltung stehen also die schlaufenförmigen Anker 28 nicht exakt radial in Bezug auf die Mittelachse A₁ des Doppelringkörpers 10 ab, sondern bilden mit dieser Mittelachse einen spitzen Winkel in Richtung auf den mittleren Bereich 16 des Doppelringkörpers 10.

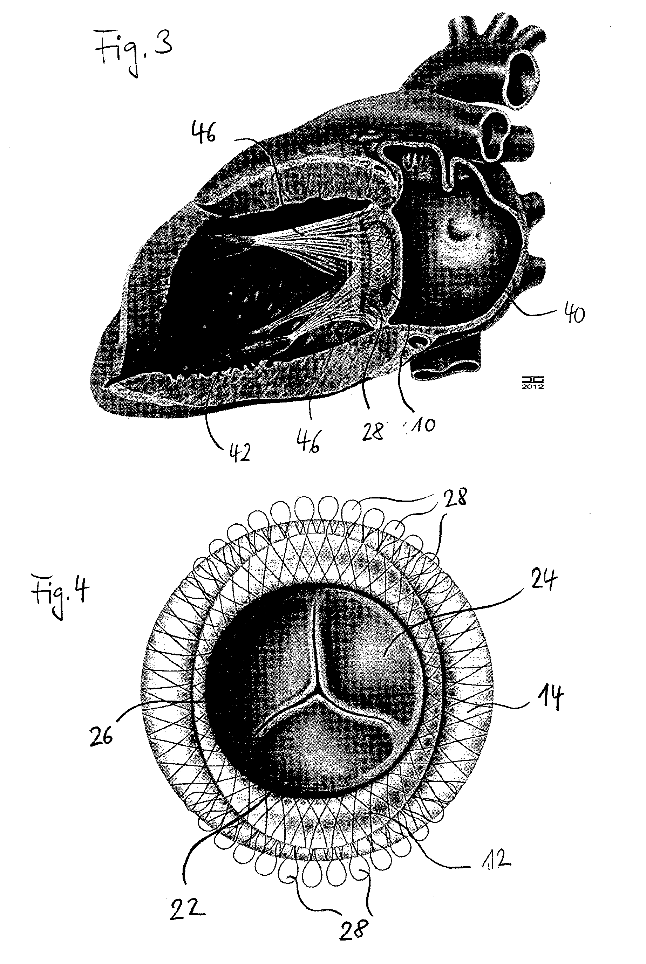

Figur 3 zeigt eine Klappenprothese gemäß den Figuren 1 und 2 im Einbauzustand zwischen Vorhof 40 und Kammer 42 eines Herzens. Der Doppelringkörper 10 ist mit seinem mittleren Bereich 16 reduzierten Durchmessers genau auf dem Annulus 44 zwischen Vorhof 40 und Kammer 42 positioniert.

Die Anker 28 greifen durch die Sehnenfäden 46 und stützen sich bei diesem Ausführungsbeispiel kammerseitig am Annulus 44 ab.

Figur 4 zeigt die Klappenprothese in axialer Draufsicht mit den Herzklappensegeln 22, 24, 26 im Schließzustand. In den Figuren sind gleiche Teile mit gleichen Bezugszeichen versehen.

### Bezugszeichenliste

- 10: Doppelringkörper
- 12: ventrikelseitiger Ring
- 14: atriumseitiger Ring
- 16: mittlerer Bereich (von 10)
- 18: "oben"
- 20: "unten"
- 22: Herzklappensegel
- 24: Herzklappensegel
- 26: Herzklappensegel
- 28: Anker
- 30: linksventrikulärer Ausflusstrakt
- 32: Lateralwand
- 40: Vorhof
- 42: Kammer
- 44: Annulus
- 46: Sehnenfäden
- A₁: Mittelachse (von 10)
- Aₗ: lange Achse (von 12)
- Aₖ: kurze Achse (von 12)
- α₁: Winkel eines Sektors
- α2: Winkel eines Sektors
- β₁: Winkel eines Sektors
- β₂: Winkel eines Sektors

## Patentansprüche

1. Klappenprothese zum Ersatz einer Atrioventricularklappe des Herzes mit einem Doppelringkörper (10), an dem Herzklappensegel (22, 24, 26) befestigt sind und der einen ventrikelseitigen Ring (12) und einen atriumseitigen Ring (14) aufweist, **dadurch gekennzeichnet, dass** der ventrikelseitige Ring (12) ovale Form hat.

2. Klappenprothese nach Anspruch 1, **dadurch gekennzeichnet, dass** an dem ventrikelseitigen Ring (12) im Wesentlichen radial nach außen stehende Anker (28) angeordnet sind.

3. Klappenprothese nach Anspruch 2, **dadurch gekennzeichnet, dass** Anker (28) nur in Sektoren des Ringes (12) vorgesehen sind, die nicht im Bereich eines Ausflusstraktes einer Herzkammer liegen.

4. Klappenprothese nach Anspruch 2, **dadurch gekennzeichnet, dass** die Anker (28) nur in Sektoren des ventrikelseitigen ovalen Rings (12) angeordnet sind, durch deren Winkel (α₁, α₂) die längere Achse (Aₗ) des Rings (12) verläuft.

5. Klappenprothese nach Anspruch 4, **dadurch gekennzeichnet, dass** die Winkel (α₁, α₂) der Sektoren kleiner sind als 160°, insbesondere kleiner als 120°.

6. Klappenprothese nach einem der Ansprüche 2 bis 5, **dadurch gekennzeichnet, dass** die Anker (28) schlaufenförmig sind.

7. Klappenprothese nach einem der vorhergehenden Ansprüche, **dadurch gekennzeichnet, dass** der Doppelringkörper zwischen dem ventrikelseitigen Ring (12) und dem atriumseitigen Ring (14) einen axial mittleren Bereich (16) hat, dessen Durchmesser geringer ist als die Durchmesser des ventrikelseitigen Ringes (12) und des atriumseitigen Ringes (14).

8. Klappenprothese nach einem der vorhergehenden Ansprüche, **dadurch gekennzeichnet, dass** der Durchmesser des ventrikelseitigen Ringes (12) geringer ist als der Durchmesser des atrialseitigen Ringes (14).

9. Klappenprothese nach einem der vorhergehenden Ansprüche, **dadurch gekennzeichnet, dass** der Doppelringkörper (10) adaptiert ist, bei Einsatz im Klappenannulus des Herzens keine den Klappenanulus wesentlich aufweitende radiale Spreizkraft zu erzeugen.

10. Klappenprothese nach einem der vorhergehenden Ansprüche, **dadurch gekennzeichnet, dass** der Doppelringkörper (10) aus einem Geflecht geformt ist, insbesondere aus einem Material mit Formgedächtnis.

11. Klappenprothese nach einem der vorhergehenden Ansprüche, **dadurch gekennzeichnet, dass** der Doppelringkörper (10) ballonexpandierbar ist.

12. Klappenprothese nach einem der Ansprüche 1 bis 10, **dadurch gekennzeichnet, dass** der Doppelringkörper (10) selbstexpandierbar ist.
